Europäisches Patentamt

European Patent Office

Office européen des brevets

(19)

(11) Veröffentlichungsnummer: **0 034 156**
**B1**

(12) # EUROPÄISCHE PATENTSCHRIFT

(45) Veröffentlichungstag der Patentschrift:
14.11.84

(51) Int. Cl.³: **G 01 N 21/31**

(21) Anmeldenummer: 80901541.5

(22) Anmeldetag: 12.08.80

(86) Internationale Anmeldenummer:
**PCT/DE 80/00119**

(87) Internationale Veröffentlichungsnummer:
**WO 81/00622 (05.03.81 Gazette 81/6)**

(54) **VERFAHREN UND VORRICHTUNG ZUR BESTIMMUNG VON GLUCOSE IM SERUM ODER IM HARN.**

(30) Priorität: 23.08.79 DE 2934190

(43) Veröffentlichungstag der Anmeldung:
26.08.81 Patentblatt 81/34

(45) Bekanntmachung des Hinweises auf die Patenterteilung:
14.11.84 Patentblatt 84/46

(84) Benannte Vertragsstaaten:
DE FR GB SE

(56) Entgegenhaltungen:
EP - A - 0 012 492
FR - A - 2 341 866
US - A - 3 405 268
US - A - 3 459 951
US - A - 3 675 019
US - A - 4 044 257

Chemical Abstracts, Band 87, 1977, (Columbus, Ohio,
US), H. FRYE u.a., "A rapid, inexpensive, ibfrared
screening method for relevated serum triglycerides",
siehe Seite 247, insgesamt, Zusammenfassung Nr.
80.696R, siehe das ganze Dokument
Applied Physics, Band 7, 1975, (Berlin, DE) G. KRAUS,
"Infrared absorption spectroscopy of aqueous solutions
with a CO2 laser", siehe Seiten 287-293, siehe i.B.
Seiten 288, 290-291

(73) Patentinhaber: **FA. CARL ZEISS,
Carl-Zeiss-Strasse 4-54, D-7082 Oberkochen (DE)**

(84) Benannte Vertragsstaaten: **DE FR SE**

(73) Patentinhaber: **CARL ZEISS-STIFTUNG HANDELND
ALS CARL ZEISS, Carl-Zeiss-Strasse 4-54,
D-7082 Oberkochen (DE)**

(84) Benannte Vertragsstaaten: **GB**

(72) Erfinder: **MÜLLER, Gerhard,
Bischof-Fischer-Strasse 106-108, D-7080 Aalen (DE)**

## Beschreibung

Die Erfindung betrifft ein Verfahren zur Bestimmung von Glucose im Serum oder auch im Harn, sowie eine Vorrichtung zur Durchführung dieses Verfahrens.

Die Bestimmung von Glucose ist von ausserordentlicher Bedeutung für die Erkennung und die Therapiekontrolle der Diabetes mellitus. Die bekannte Ermittlung über Teststreifen im Harn dient hauptsächlich zur Feststellung der Erkrankung. Obgleich ein gut eingestellter Diabetiker bei regelmässiger Urinuntersuchung mit wenigen Stichproben der Blutglucose auskommen kann, spielt für die Therapie, die Therapiekontrolle, die Einstellung des Tagesprofils usw. im wesentlichen die Bestimmung der Glucosekonzentration im Blut eine Rolle.

Die bekannten Verfahren zur Glucosebestimmung lassen sich in drei Gruppen einteilen, nämlich in biochemische, elektrochemische und spektroskopische Verfahren. Von diesen Verfahren sind die biochemischen Verfahren nur als Laborverfahren geeignet; die elektrochemischen Verfahren bieten zwar derzeit die aussichtsreichsten Ansatzpunkte für die Entwicklung von implantierbaren Glucosesensoren, sind aber in ihrer Genauigkeit und Spezifität ebenso wie die biochemischen Verfahren den spektroskopischen Verfahren unterlegen.

Die biochemischen und elektrochemischen Verfahren haben zudem den Nachteil gemeinsam, dass die zu messende Probe vor dem eigentlichen Messvorgang durch Zusatz von Chemikalien so aufbereitet werden muss, dass messtechnisch erfassbare Reaktionsprodukte gebildet werden. Damit sind kontinuierliche Messungen nicht möglich.

Im Gegensatz zu den biochemischen und elektrochemischen Verfahren wird beim spektroskopischen Verfahren das Glucosemolekül nicht verändert. Daneben gibt es noch einige unspezifische Verfahren, die praktisch nicht mehr in Gebrauch sind. So ist beispielsweise aus der DE-A-2 724 543 ein Verfahren zur Bestimmung von Glucose auf der Grundlage der seit langem bekannten, aber wegen nicht hinreichender Spezifität nur noch selten angewandten Polarimetrie bekannt, welches allenfalls unter günstigen Umständen zur Zuckerbestimmung im Harn geeignet ist.

Eine erste Möglichkeit zur Bestimmung von Stoffwechselprodukten durch spektroskopische Messung stellt die Laser-Raman-Spektroskopie dar. Die Frequenz der Erregerstrahlung liegt dabei im sichtbaren Spektralbereich. Zur Messung wird der nach Rot verschobene Teil im Spektrum des Streulichts verwendet.

Bei Messungen im Vollblut tritt bei diesem Messverfahren die Schwierigkeit auf, dass im gesamten sichtbaren Spektralbereich Blut, bedingt durch Hämoglobin und die anderen chromophoren Substanzen, eine starke Absorption zeigt, was zwar für diese Moleküle zu einer Resonanzverstärkung der Raman-Streuung führt, jedoch mit einem hohen Fluoreszenzuntergrund verbunden ist und somit die Detektion nicht resonanzverstärkter Banden erschwert, wenn nicht ganz unmöglich macht. Dieses Problem lässt sich zwar mit den heute zur Verfügung stehenden Möglichkeiten zur Anregung der Raman-Streuung mit kurzen Laserimpulsen im Subnanosekunden-Bereich lösen; jedoch ist der technische Aufwand so erheblich, dass sich damit in absehbarer Zeit für Vollblutproben kein praktisch einsetzbares Verfahren realisieren lassen wird.

Eine andere Möglichkeit, die Störung durch Fluoreszenz der Chromophore zu vermeiden, besteht darin, im infraroten Spektralbereich zu arbeiten, d.h. das Infrarot-Spektrum der Probe direkt aufzunehmen. Da aber die Probenaufbereitung, sowie die Registrierung und Auswertung des Spektrums zeitaufwendig und kompliziert ist, stellt die Infrarot-Spektroskopie in der bisher üblichen Art keine Konkurrenz zu den bestehenden anderen Verfahren dar.

Ein weiteres spektroskopisches Verfahren ist die sogenannte ATR-Spektroskopie, d.h. die Totalreflexionsspektroskopie mit quergedämpfter Welle. So ist es beispielsweise aus FR-A-2 341 866 ( = DE-A1-26 06 991) bekannt, einen $CO_2$-Laser in Verbindung mit der seit langem bekannten ATR-Spektroskopie zur Bestimmung von Glucose bzw. auch von anderen Stoffwechselprodukten zu verwenden. Dies lässt sich jedoch nur in reinen Lösungen durchführen; in Mehrkomponentensystemen oder gar im Vollblut muss dieses Verfahren aufgrund prinzipieller Schwierigkeiten scheitern.

So gilt zum Beispiel das Lambert-Beer'sche Gesetz für die ATR-Spektroskopie nur für einen Absorptionskoeffizienten im Bereich von 0,1 und kleiner sowie für einen Einfallswinkel von ungefähr 60° oder grösser, bezogen auf die im IR üblichen Materialien, und ausserdem nur bei hinreichend grossen Wellenlängen, und auch hier nur annäherungsweise für isotrope Proben. Selbst bei in vitro Messungen im Vollblut würden hier also bereits Schwierigkeiten auftreten, bedingt zum Beispiel auch durch die Proteinabsorption an den Reflexionsflächen, die das System in eine Anisotropie überführen.

Aus US-A-3 675 019 und US-A-3 405 268 ist es bekannt den Wassergehalt von Papier dadurch zu bestimmen, dass gleichzeitig bei zwei unterschiedlichen Wellenlängen die Absorption von Infrarotstrahlung durch die Probe gemessen und der Quotient aus den bei diesen beiden Wellenlängen gemessenen Absorptionswerten gebildet wird. Eine Wellenlänge ist dabei so ausgewählt, dass die Absorption vom Wassergehalt der Probe abhängt, die andere Wellenlänge ist so gewählt, dass die Absorption vom Wassergehalt nicht beeinflusst wird. Die Auswahl der Wellenlängen ist hier sehr einfach, da nur das Absorptionsverhalten von Wasser zu berücksichtigen ist.

Aus US-A-4 044 257 ist ein in Verbindung mit einem Chromatographen verwendeter absorptionsspektrographischer IR-Detektor bekannt, welcher folgende Elemente aufweist: eine Infrarotstrahlungsquelle zur Erzeugung eines Infrarotstrahlungsbündels, das aus Infrarotstrahlung einer ersten Wellenlänge $\lambda_1$ und einer zweiten Wel-

lenlänge $\lambda_2$ besteht, eine Infrarotstrahlungsdetektoreinrichtung, einen im Infrarotstrahlengang zwischen der Quelle und dem Detektor positionierten Probenträger und eine der Detektoreinrichtung nachgeschaltete Divisions-Schaltung zur Bildung des Quotienten aus den ermittelten Absorptionswerten, wobei die Vorrichtung zur simultanen Messung der Absorptionswerte der Infrarotstrahlung bei den beiden unterschiedlichen Wellenlängen ($\lambda_1$, $\lambda_2$) ausgebildet ist.

Aus EP-A1-0 012 492 ist es bekannt den Fettgehalt einer Probe durch Absorption von Infrarotstrahlung aus einem Wellenlängenbereich zu messen, der für gesättigte Kohle-Wasserstoff-Verbindungen charakteristisch ist. Um störende Effekte, wie z.B. eine Verschmutzung der Küvette kompensieren zu können, erfolgt gleichzeitig eine Absorptionsmessung bei einer benachbarten Wellenlänge und es wird der Quotient aus den beiden gemessenen Absorptionswerten gebildet.

Aus der ZS «Applied Physics», Band 7, 1975 (Berlin DE), S. 287–293 ist aus dem Aufsatz «Infrarot Absorption Spectroscopy of aqueous solutions with $CO_2$ Laser» von G. Kraus ein Verfahren zur Bestimmung von Glucose im Serum bekannt, bei dem die Absorption von Infrarotstrahlung durch eine Probe und eine Referenzsubstanz gemessen und aus den Messwerten die Probenkonzentration rechnerisch ermittelt wird.

Schliesslich ist in der ZS «Chemical Abstracts» Bd. 87, 1977 auf S. 247 der Abstract No. 80696 veröffentlicht, der kurz über eine Arbeit von Frye u.a. über die Untersuchung von menschlichem Serum auf erhöhten Triglycerid-Spiegel berichtet. Als Messwert wird hier die Differenz der bei zwei Infrarot-Wellenlängen, nämlich 1740 und 1725 cm$^{-1}$ gewonnenen Absorptionswerte bestimmt.

Die vorliegende Erfindung befasst sich nun mit der Bestimmung von Glucose im Serum oder im Harn, d.h. in einem Mehrkomponenten-Gemisch.

Es ist dabei die Aufgabe der Erfindung ein Verfahren zu schaffen, das ohne Probenvorbehandlung eine schnelle, quantitative und reproduzierbare Messung von Glucose im natürlichen biologischen Milieu ermöglicht, die ein normiertes Messsignal erzeugt.

Diese Aufgabe wird von dem in Anspruch 1 beschriebenen Verfahren gelöst.

Bei dem erfindungsgemässen Verfahren liegt die erste Wellenlänge $\lambda_1$ in einem «quasi-isobestischen» Bereich, während die andere Wellenlänge $\lambda_2$ im Bereich einer substanzspezifischen Absorptionsbande liegt, und zwar so, dass bei dieser letzten Wellenlänge eine Absorptionsänderung nur durch eine Konzentrationsänderung der zu bestimmenden Glucose verursacht wird.

Beim erfindungsgemässen Verfahren wird die zur Messung dienende charakteristische Wellenlänge $\lambda_2$ in einem Bereich gewählt, in dem die störenden Komponenten des Gemisches vor allem Heparin und Wasser eine möglichst minimale Änderung der Absorption, Glucose dagegen eine möglichst maximale Änderung der Absorption bei einer Konzentrationsänderung zeigen.

Das Verfahren nach der Erfindung kann in der Weise durchgeführt werden, dass man die Absorption bei den beiden unterschiedlichen Wellenlängen mittels Transmissions-Spektroskopie oder mittels Reflexions-Spektroskopie, beispielsweise ATR-Spektroskopie, bestimmt. Transmissionsmessungen haben gegenüber Messungen mittels ATR-Spektroskopie den Vorteil, dass das Lambert-Beer'sche Gesetz hier gültig ist und dass sie für die Erzielung quantitativer Messwerte durch biochemische Absolutbestimmung kalibriert werden können.

Die zu messende Probe liegt zweckmässig als Ausstrich bzw. Film auf einem Wegwerf- oder Einwegprobenträger aus Kunststoff vor. Das Trägermaterial ist so gewählt, dass es im Bereich der ersten und zweiten Wellenlänge nur eine geringe Eigenabsorption hat. Der Probenträger kann in bekannter Weise als ebener Objektträger, gegebenenfalls aber auch als Durchfluss- oder Trogküvette ausgebildet sein.

Eine Vorrichtung zur Durchführung des erfindungsgemässen Verfahrens zeichnet sich aus durch die im Anspruch 7 angegebenen Merkmale.

Die Infrarotstrahlungsquelle kann einen starken Kontinuumstrahler oder einen, bzw. mehrere Laser enthalten, wobei Gas- oder Festkörperlaser Verwendung finden können. Zur Aussonderung der beiden zur Messung verwendeten Wellenlängen können Interferenzfilter oder entsprechend ausgebildete Strahlteiler vorgesehen sein.

Vorteilhaft ist es empfangsseitig nur einen Detektor zu verwenden und die Strahlenbündel unterschiedlicher Wellenlänge durch unterschiedliche Modulation unterscheidbar zu machen.

Durch die Quotientenbildung der Absorptionswerte, die man für die beiden Wellenlängen misst, erhält man ein normiertes Messsignal, wobei die Probe selbst als Referenz dient.

Weitere Ausgestaltungen der Erfindung sind in den Unteransprüchen wiedergegeben.

Die Erfindung wird im folgenden anhand der Figuren 1 bis 12 der beigefügten Zeichnungen näher erläutert. Es zeigen:

Fig. 1 ein Infrarotabsorptionsspektrum von Wasser;

Fig. 2 ein Infrarotabsorptionsspektrum von Heparin;

Fig. 3 ein Infrarotabsorptionsspektrum von D-Glucose;

Fig. 4 ein Infrarotabsorptionsspektrum von getrocknetem menschlichem Vollblut, bei dem der Glucosegehalt im normal-physiologischen Bereich liegt;

Fig. 5 ein Infrarotabsorptionsspektrum von getrocknetem menschlichem Vollblut, das mit D-Glucose soweit angereichert ist, dass der Glucose-Gehalt im pathologischen Bereich liegt;

Fig. 6 ein erstes Ausführungsbeispiel einer erfindungsgemässen Vorrichtung;

Fig. 7 ein zweites Ausführungsbeispiel einer Vorrichtung;

Fig. 8 ein drittes Ausführungsbeispiel der Erfindung mit einer abgewandelten Infrarotstrahlungsquelle;

Fig. 9 ein viertes Ausführungsbeispiel der Erfindung;

Fig. 10 ein fünftes Ausführungsbeispiel der Erfindung;

Fig. 11 Durchlasskurven zweier Einband-Interferenzfilter, wie sie in den Ausführungsbeispielen nach den Fig. 6, 7 und 9 Verwendung finden; und eine Durchlasskurve eines Doppelband-Interferenzfilters, wie es im Ausführungsbeispiel nach der Fig. 8 vorgesehen ist; und

Fig. 12 die Durchlasskurven eines wellenlängenselektiven Strahlenteilers, wie er in den Ausführungsformen nach den Fig. 7 und 9 vorgesehen ist.

Eine wesentliche Schwierigkeit bei der Bestimmung von Glucose im Vollblut oder Harn besteht darin, dass die Körperflüssigkeiten zu einem hohen Prozentsatz aus Wasser bestehen. Wasser hat aber im Bereich der Glucoseabsorption im Infraroten einen Absorptionskoeffizienten von 700 cm$^{-1}$. Glucose hat dagegen in diesem Bereich lediglich einen Absorptionskoeffizienten von ca. 0,1 cm$^{-1}$. Durch den Einsatz von Lasern anstelle von konventionellen Lichtquellen lässt sich dieses Problem reduzieren.

Beim Verfahren nach der Erfindung wird die Absorption der Probe bei zwei verschiedenen Wellenlängen simultan bestimmt. Die beiden Wellenlängen liegen dabei so dicht beieinander, dass dispersive Effekte klein zu halten sind. Dies ermöglicht eine Messung im biologischen Milieu ohne komplizierte und langwierige Probenvorbehandlung.

Die erste Wellenlänge $\lambda_1$ ist so gewählt, dass bei Glucose-Konzentrationsänderungen in der Probe keine oder nur eine vernachlässigbar kleine Änderung der Absorption auftritt, d.h. $\lambda_1$ liegt im «isosbestischen Punkt» oder in einem «quasi-isosbestischen» Bereich.

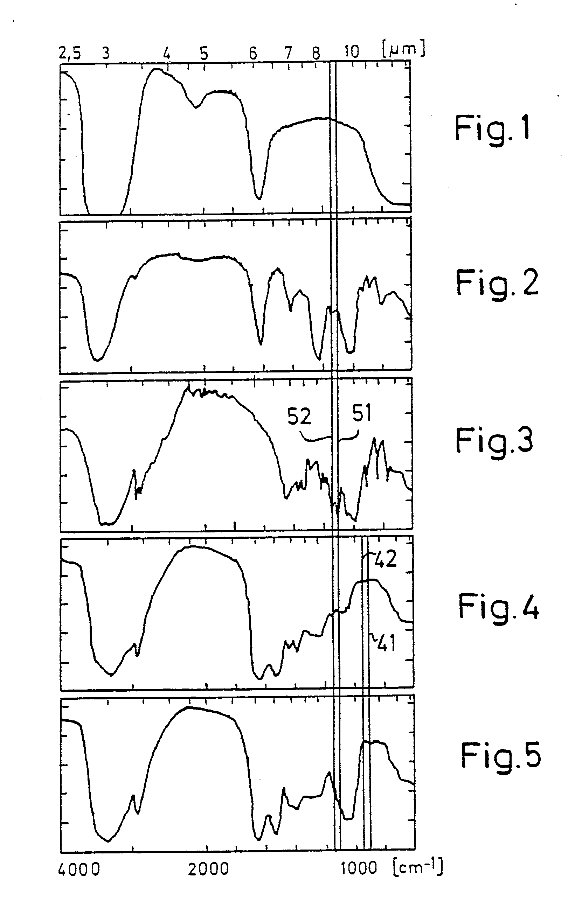

Ein solcher quasi-isobestischer Bereich ist in den Figuren 4 und 5 durch die Linien 41 und 42 verdeutlicht. Man erkennt, dass in diesem Bereich bei einer Änderung der Glucose-Konzentration nur eine vernachlässigbar kleine Änderung der Absorption auftritt. Diese Absorption entspricht also der Grundabsorption der Probe und ist zur Normierung des Messsignals geeignet.

Die Linie 41 entspricht dem Wert 940 cm$^{-1}$, die Linie 42 entspricht dem Wert 950 cm$^{-1}$.

Die zweite Wellenlänge $\lambda_2$ ist so gewählt, dass sie auf einer substanzspezifischen Absorptionsbande liegt. Diese Bedingung erfüllen Wellenlängen im Bereich zwischen den beiden Wellenlängen, die in den Figuren 1–5 mit 51 und 52 bezeichnet sind. Linie 51 entspricht einem Wert $\lambda_2$ von 1090 cm$^{-1}$, Linie 52 entspricht dem Wert 1095 cm$^{-1}$. Man erkennt aus den Figuren 3 und 2, dass der angegebene Bereich auf einer Absorptionsbande der Glucose, zugleich aber im Bereich minimaler Absorption bei Heparin liegt. Das Muccopolysaccharid Heparin ist im Vollblut zu einem relativ hohen Prozentsatz in den basophilen Leukozyten vorhanden. Im Normalfall ist der Einfluss nicht allzu hoch, etwa 0,5%, jedoch kann in pathologischen Grenzsituationen mit einem erhöhten Leukozytenanteil oder auch nach Verabreichung von Heparin als Antikoagulanz eine empfindliche Störung der Glucosemessung entstehen. Durch die dargestellte Wahl der Wellenlänge $\lambda_2$ ist eine Störung der Messung durch Heparin in der Probe vermieden.

Verwendet man als Strahlungsquelle einen $CO_2$-Laser, so entsprechen die $\lambda_2$-Linien den Laserlinien R (40) bis R (52) und der $\lambda_1$-Wellenlängenbereich liegt zwischen den $CO_2$-Laserlinien P (14) bis P (26). Die Wellenlängenselektion wird zweckmässig durch geeignete Interferenzfilter vorgenommen, die in diesen Bereichen nach dem Stand der Technik mit einer Halbwertsbreite von etwa 5 cm$^{-1}$ hergestellt werden können. Statt eines $CO_2$-Lasers kann auch ein Halbleiterlaser, beispielsweise ein $Pb_{1-x}$-$Sn_x$-Te- oder ein Raman-Laser, oder auch ein Kontinuumstrahler mit Frequenzselektion verwendet werden. Die Detektion des Messsignals erfolgt mit den nach dem Stand der Technik üblichen Verfahren und Vorrichtungen.

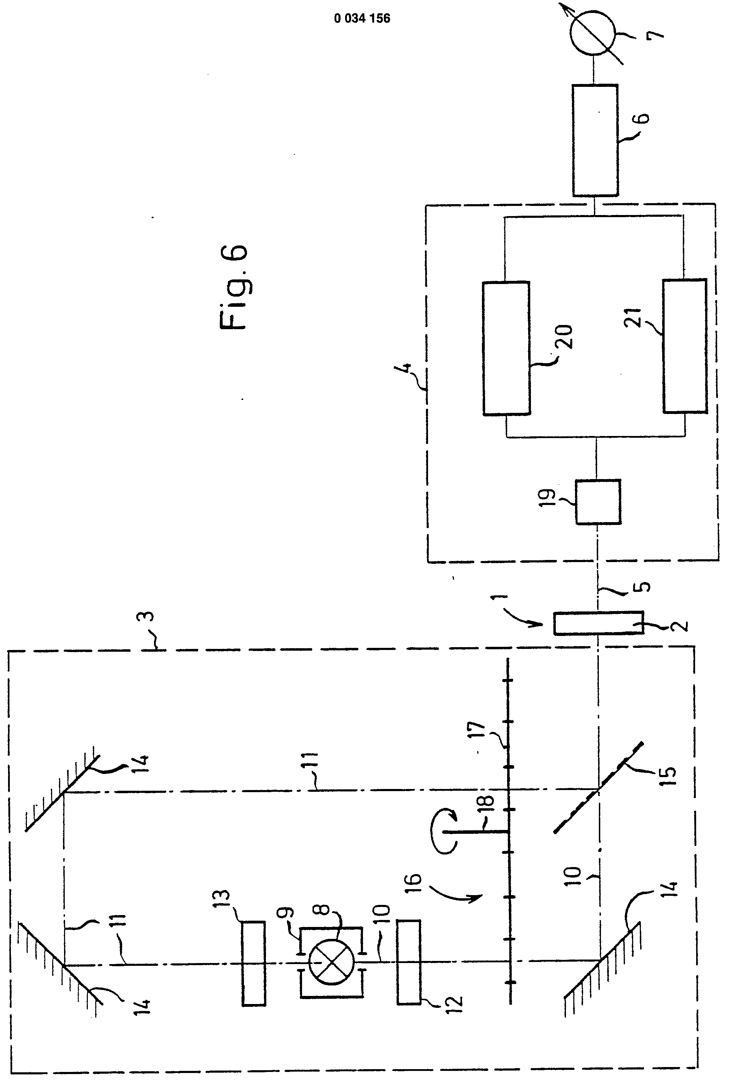

Die in den Fig. 6 bis 10 dargestellten Ausführungsbeispiele von Vorrichtungen zur Bestimmung von Stoffwechselprodukten können sowohl zur Transmissionsspektroskopie als auch zur ATR-Totalreflexion-Spektroskopie mit quergedämpften Infrarotlichtwellen verwendet werden. Dabei ist der nur schematisch angedeutete Probenträger 1, in oder auf dem die Probe 2 vorgesehen ist, vorzugsweise entweder als Objektträgerplättchen oder als Küvette aus zum Beispiel einem Copolymer aus Polyäthylen und Polypropylen gefertigt, je nachdem ob die Probe 1 senkrecht oder horizontal durchstrahlt wird.

Jedes der gezeigten Ausführungsbeispiele weist eine Infrarotstrahlungsquelle 3 und eine Detektoreinrichtung 4 auf. Im Infrarotstrahlungsbündel 5, das den Strahlengang zwischen der Quelle 3 und dem Detektor 4 bildet, befindet sich der Probenträger 1 mit der zu messenden Probe 2.

Die Infrarotstrahlungsquelle 3 ist so ausgebildet, dass sie das Infrarotstrahlungsbündel 5 erzeugt, welches aus Infrarotstrahlung einer ersten und einer zweiten Wellenlänge $\lambda_1$ bzw. $\lambda_2$ besteht. Die Detektoreinrichtung 4 ist so ausgebildet, dass sie die Absorptions- bzw. Intensitätswerte der Infrarotstrahlung der unterschiedlichen Wellenlängen $\lambda_1$ und $\lambda_2$ gesondert misst. Der Detektoreinrichtung 4 ist eine Divisionsschaltung 6 nachgeschaltet, der die beiden von der Detektoreinrichtung 4 ermittelten Signale $I_1$ und $I_2$ zugeführt werden und die den Quotienten $Q = I_2/I_1$ bildet, worin $I_2$ der der Wellenlänge $\lambda_2$ entsprechende Absorptions- bzw. Intensitätswert ist, während $I_1$ der der Wellenlänge $\lambda_1$ entsprechende Absorptions- bzw. Intensitätswert ist. Dieser Divisionsschaltung 6 ist eine Anzeige- und/oder Aufzeichnungseinrichtung 7 nachgeschaltet, die beispielsweise als Schreiber, Drucker, Digital- oder Analoganzeigegerät o.dgl. ausgebildet sein kann.

Im Ausführungsbeispiel der Fig. 6 weist die Infrarotstrahlungsquelle 3 einen starken Kontinuumsstrahler 8 auf, wie beispielsweise einen Globar- oder Nernst-Stift. Mittels einer Blende 9 wird ein erstes und zweites Infrarotstrahlungsbündel 10 bzw. 11 ausgeblendet. Zur Selektierung der beiden Wellenlängen $\lambda_1$ und $\lambda_2$ aus dem Kontinuumspektrum der Strahlungsquelle 8 ist im Strahlengang des Teilstrahles 10 ein erstes Interferenzfilter 12 angeordnet, das im wesentlichen nur Strahlung der Wellenlänge $\lambda_1$ durchlässt, während im Strahlengang des zweiten Teilstrahles 11 ein zweites Interferenzfilter 13 angeordnet ist, das im wesentlichen nur Strahlung der Wellenlänge $\lambda_2$ durchlässt.

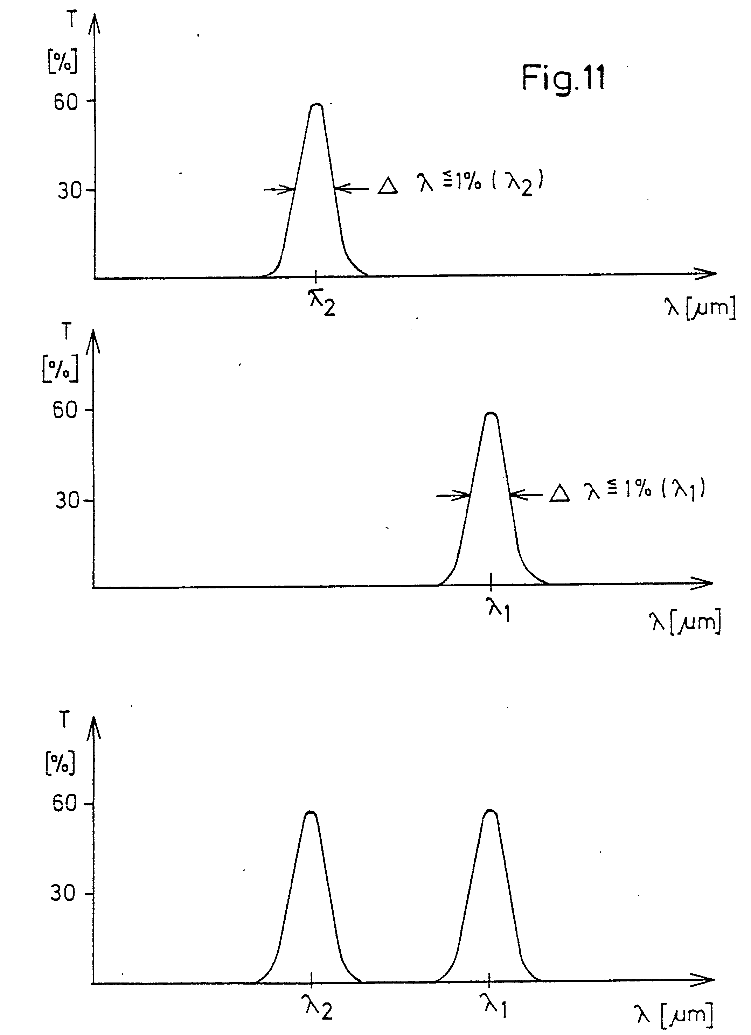

Der prinzipielle Verlauf der Durchlassfunktion der Interferenzfilter 12, 13 ist im oberen und mittleren Teil der Fig. 11 dargestellt, worin die Transparenz T in Prozent über der Wellenlänge $\lambda$ in $\mu$m aufgetragen ist. Die Halbwertsbreite $\Delta\lambda$ sollte kleiner oder gleich 1% der jeweiligen durchzulassenden Wellenlänge $\lambda_1$ bzw. $\lambda_2$ sein.

Die beiden Teilstrahlen 10, 11 werden über Spiegel 14 und einen wellenlängenselektiven Strahlteiler 15 zu einem einzigen Infrarotstrahlungsbündel 5 vereinigt, nachdem sie vorher durch einen Chopper 16 mit unterschiedlichen Frequenzen $f_1$ (Modulationsfrequenz des Infrarotstrahlungsbündels 10) bzw. $f_2$ (Modulationsfrequenz des Infrarotstrahlungsbündels 11) moduliert worden sind. Dass sich unterschiedliche Frequenzen ergeben, ist dadurch angedeutet, dass die Unterbrecherscheibe 17 des Choppers 16 das Infrarotstrahlungsbündel 11 an einer der Rotationsachse 18 des Choppers 16 näher gelegenen Stelle schneidet als das Infrarotstrahlungsbündel 10 und das Chopperblatt für die beiden Bündel (10, 11) eine unterschiedliche Zahl von Segmenten hat.

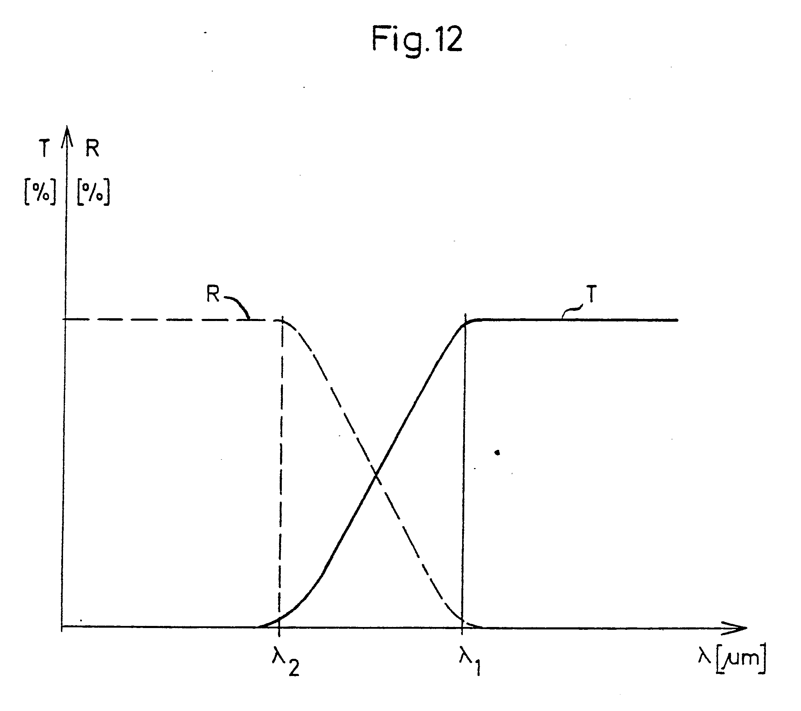

Die Durchlassfunktion des wellenlängenselektiven Strahlenteilers 15 ist in Fig. 12 dargestellt, worin die Transparenz T bzw. die Reflexionsfähigkeit R, jeweils in Prozent, über der Wellenlänge in $\mu$m aufgetragen und die Wellenlängen $\lambda_1$ und $\lambda_2$ eingezeichnet sind.

Das Strahlungsbündel 5 tritt durch die Probe 2, die zum Beispiel ein Ausstrich eines Bluttropfens auf einem als Objektträger ausgebildeten Probenträger 1 sein kann. Dieser kann beispielsweise aus einem Copolymer aus Polyäthylen und Polypropylen bestehen. Nach Durchtritt durch die Probe 2 gelangt das Infrarotstrahlungsbündel 5 in die Detektoreinrichtung 4, und trifft hier auf einen einzigen Detektor 19 auf, der, in dem jeweiligen Spektralbereich, in dem sich die Wellenlängen $\lambda_1$ und $\lambda_2$ befinden, nahezu gleiche Empfindlichkeit besitzt. Beispielsweise kann der Detektor 19 ein pyroelektrischer Empfänger, wie etwa ein Triglycinsulfat-Kristall sein, der abgekürzt auch als TGS-Kristall bezeichnet wird. Diese nahezu gleiche Empfindlichkeit ist notwendig, damit die nachgeschaltete Elektronik mit gleichen Zeitkonstanten betrieben werden kann. Das Ausgangssignal des Detektors 19 wird zwei parallelen Lock-in-Verstärkern 20, 21 (phasenempfindliche Gleichrichter mit gegebenenfalls nachgeschaltetem Verstärker) zugeführt, von denen einer auf die Modulationsfrequenz $f_1$ des Infrarotstrahlungsanteils der Wellenlänge $\lambda_1$ und der andere auf die Modulationsfrequenz $f_2$ des Infrarotstrahlungsanteils der Wellenlänge $\lambda_2$ abgestimmt ist. Am Ausgang des Lock-in-Verstärkers 20 erhält man den oben erwähnten Intensitätswert $I_1$ bzw. ein ihm proportionales Signal, und am Ausgang des Lock-in-Verstärkers 21 steht der oben genannte Intensitätswert $I_2$ bzw. ein ihm proportionales Signal zur Verfügung. Diese beiden Signale werden in die nachgeschaltete Divisionsschaltung 6 eingegeben, die hieraus das normierte konzentrationsproportionale Signal $Q = I_2/I_1$ erzeugt.

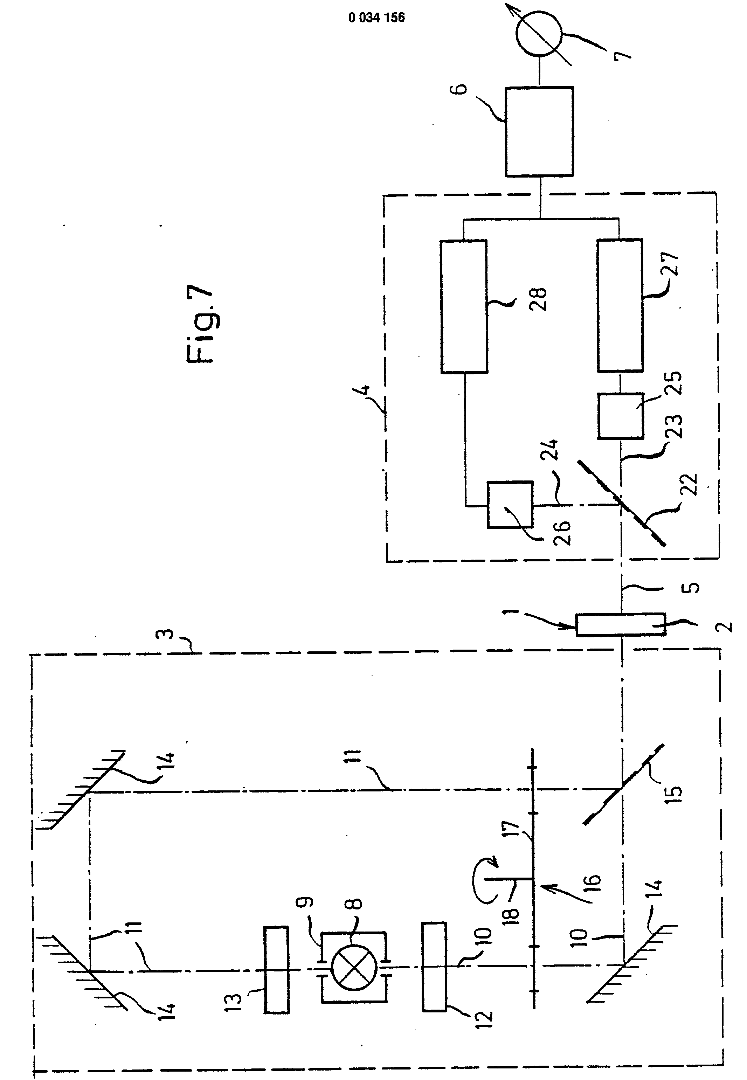

Im Ausführungsbeispiel der Fig. 7 ist der Chopper 16 so angeordnet und ausgebildet, dass er beide Teilstrahlen 10 und 11 mit der gleichen Chopfrequenz f moduliert. Infolgedessen ist in der zugehörigen Detektoreinrichtung 4 ein zweiter wellenlängenselektiver Strahlenteiler 22 vorgesehen, der das darauf auftreffende einzige Infrarotstrahlungsbündel 5 in ein erstes Teilbündel 23 der Wellenlänge $\lambda_1$ und ein zweites Teilbündel 24 der Wellenlänge $\lambda_2$ aufteilt. Das erste Teilbündel 23 gelangt auf einen Detektor 25, und das zweite Teilbündel 24 trifft auf einen zweiten Detektor 26 auf. Diese Detektoren 25, 26 können von der gleichen Art wie der Detektor 19 der Fig. 6 sein.

Die den beiden Detektoren 25, 26 nachgeschalteten Lock-in-Verstärker 27 bzw. 28, die beide auf die Modulationsfrequenz f abgestimmt sind, erzeugen wiederum an ihren Ausgängen Intensitätswerte $I_1$ bzw. $I_2$, aus denen der oben erwähnte Quotient Q in der Divisionsschaltung 6 gebildet wird.

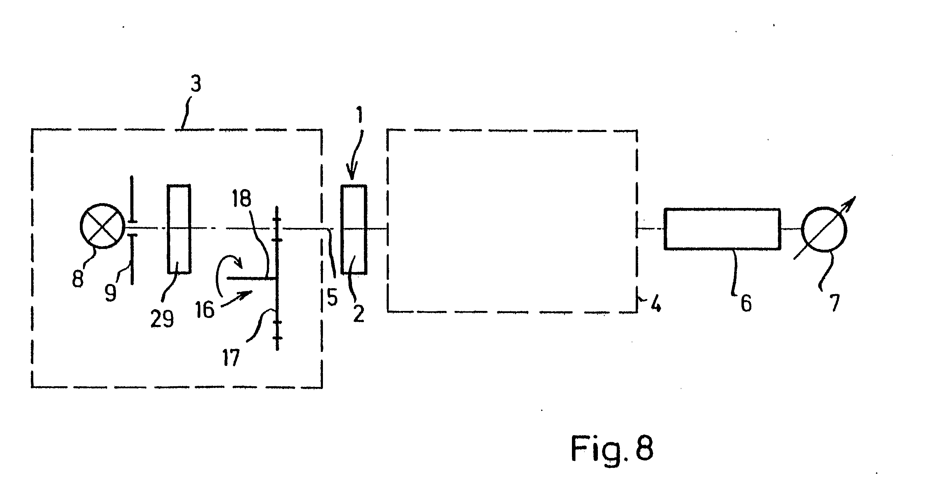

Fig. 8 zeigt ein Ausführungsbeispiel, bei dem aus der Strahlung der ebenfalls als Kontinuumsstrahler ausgebildeten Strahlungsquelle 8 mittels der Blende 9 nur ein einziges Strahlungsbündel, nämlich das Infrarotstrahlungsbündel 5 ausgeblendet wird. Dieses Bündel tritt durch einen Doppelband-Interferenzfilter 29 und die Unterbrecherscheibe 17 eines Choppers 16 und durchsetzt dann die Probe 2. Die Durchlasskurve dieses Doppelband-Interferenzfilters 29 ist im unteren Teil der Fig. 11 gezeigt und stellt, wie ein Vergleich mit dem mittleren und oberen Teil der Fig. 11 ergibt, eine Kombination der Durchlasskurven der beiden Interferenzfilter 12 und 13 dar, die in den Ausführungsbeispielen der Fig. 6 und 7 verwendet werden.

Die Detektoreinrichtung 4 in Fig. 8 kann so ausgebildet sein, wie in Fig. 7 dargestellt.

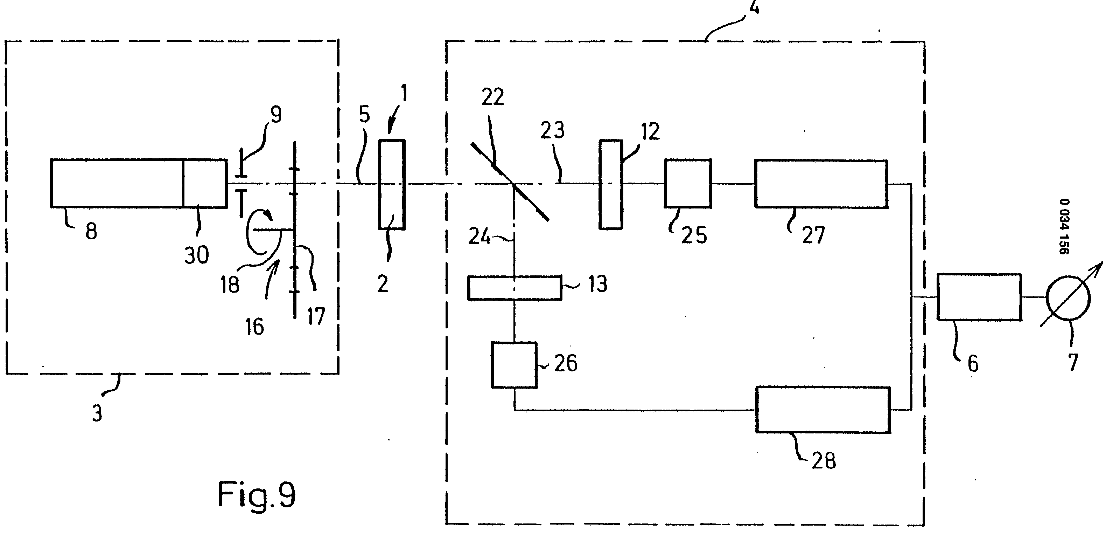

Fig. 9 zeigt ein Ausführungsbeispiel, bei dem die Infrarotstrahlungsquelle 3 als Lichtquelle einen Laser 8, zum Beispiel einen $CO_2$-Laser oder einen Raman-Laser, mit Mehrlinienemission aufweist. Dieser ist mit einer Strahlaufweitungsvorrichtung 30 versehen. Mittels der Blende 9 wird ein einziges Strahlungsbündel, nämlich das Infrarotstrahlungsbündel 5, ausgeblendet, das, bevor es durch die Probe 2 tritt, mittels eines Choppers 16 mit einer Chop-Frequenz f moduliert wird.

Die Detektoreinrichtung 4 ist im wesentlichen so aufgebaut, wie in Fig. 7 dargestellt; jedoch ist zum Zwecke der Wellenlängenselektion ein erstes Interferenzfilter 12 im Strahlengang des ersten Teilbündels 23 zwischen dem wellenlängenselektiven Strahlenteiler 22 und dem ersten Detektor 25 angeordnet, so dass letzterer nur Infrarotstrahlung der Wellenlänge $\lambda_1$ erhält, während im Strahlengang des zweiten Teilbündels 24 zwischen dem wellenlängenselektiven Strahlenteiler 23 und dem zweiten Infrarotstrahlungsdetektor 26 ein zweites Interferenzfilter 13 vorgesehen ist, das nur Strahlung der Wellenlänge $\lambda_2$ durchlässt.

Das Ausführungsbeispiel nach Fig. 9 kann auch so abgewandelt sein, dass durch Aufteilung und Wiedervereinigung des aus der Strahlaufweitungsvorrichtung 30 austretenden Strahlungsbündels unter Selektion der Wellenlängen $\lambda_1$ und $\lambda_2$ und einer Zweifrequenzmodulation mittels eines Choppers 16 entsprechend der Ausführungsform nach Fig. 6 eine Detektoreinrichtung 4 der in Fig. 6 gezeigten Art verwendet werden kann, die nur einen einzigen Detektor 19 erfordert.

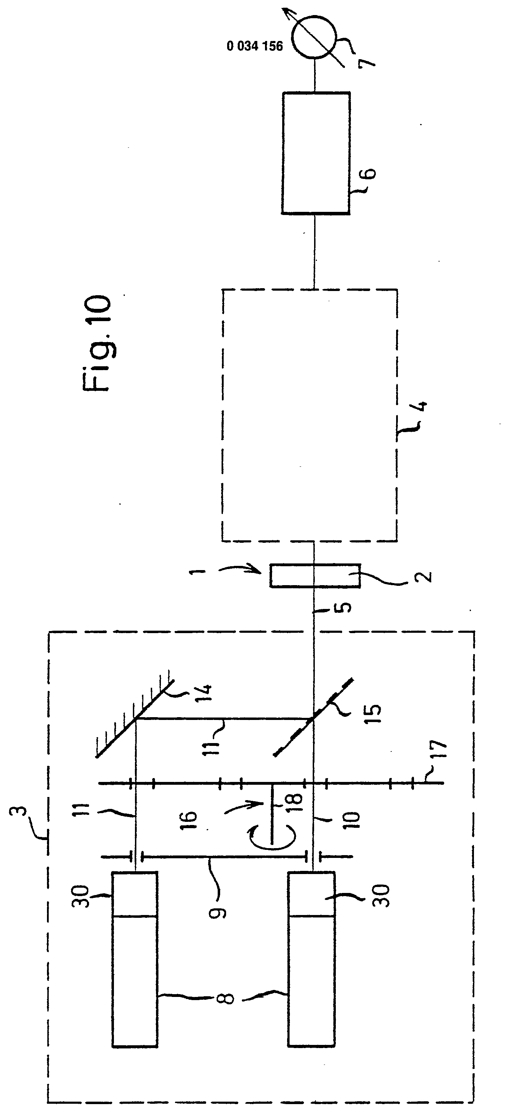

In Fig. 10 ist ein Ausführungsbeispiel gezeigt, in dem zwei monochromatische Laser 8 vorgesehen sind, von denen der eine die Emissionswellenlänge $\lambda_1$ und der andere die Emissionswellenlänge $\lambda_2$ hat. Diese Laser 8 können beispielsweise $Pb_{1-x}Sn_xTe$-Laser sein, so dass nach Durchgang der Laserstrahlungen durch die Strahlaufweitungsvorrichtung 30 mittels der Blende 9 ein erstes Teilbündel 10 erzeugt wird, das nur Infrarotstrahlung der Wellenlänge $\lambda_1$ enthält, sowie ein zweites Teilbündel 11, das nur Infrarotstrahlung der Wellenlänge $\lambda_2$ enthält. Diese beiden Teilbündel werden in ähnlicher Weise wie in Fig. 6 mit zwei unterschiedlichen Chop-Frequenzen $f_1$ bzw. $f_2$ moduliert und durch einen Spiegel 14 und einen wellenlängenselektiven Strahlenteiler 15 zu dem gemeinsamen Infrarotstrahlungsbündel 5 vereinigt. Die Detektoreinrichtung 4 kann bei diesem Ausführungsbeispiel von der in Fig. 6 gezeigten Art sein, sie kann jedoch auch so ausgebildet sein, wie in Fig. 7 gezeigt ist, sofern man die beiden Teilbündel 10, 11 in Fig. 10 mit der gleichen Chop-Frequenz f moduliert.

**Patentansprüche**

1. Verfahren zur Bestimmung von Glucose im Serum oder im Harn, bei dem bei zwei unterschiedlichen Wellenlängen ($\lambda_1$, $\lambda_2$) die Absorption von Infrarotstrahlung durch eine Probe gemessen wird, dadurch gekennzeichnet, dass die Absorption der Probe simultan bei einer ersten Wellenlänge ($\lambda_1$) im Bereich 940 bis 950 cm$^{-1}$ und einer zweiten Wellenlänge ($\lambda_2$) im Bereich 1090 bis 1095 cm$^{-1}$ gemessen und der Quotient aus den bei diesen beiden Wellenlängen ($\lambda_1$, $\lambda_2$) gemessenen Absorptionswerten gebildet wird, wobei die Glucose-Konzentration dem bei der zweiten Wellenlänge ($\lambda_2$) gemessene Absorptionswert proportional ist, während der bei der ersten Wellenlänge ($\lambda_1$) gemessene Absorptionswert praktisch unabhängig von dieser Konzentration ist.

2. Verfahren nach Anspruch 1, dadurch gekennzeichnet, dass die Absorption bei den beiden unterschiedlichen Wellenlängen ($\lambda_1$, $\lambda_2$) mittels Transmissions-Spektroskopie bestimmt wird.

3. Verfahren nach Anspruch 1, dadurch gekennzeichnet, dass die Absorption bei den beiden unterschiedlichen Wellenlängen ($\lambda_1$, $\lambda_2$) mittels Reflexions-Spektroskopie bestimmt wird.

4. Verfahren nach einem der Ansprüche 1 bis 3, dadurch gekennzeichnet, dass die Probe (2) als Ausstrich bzw. Film vorgesehen ist.

5. Verfahren nach Anspruch 4, dadurch gekennzeichnet, dass als Probenträger (1) ein Wegwerf- bzw. Einwegprobenträger aus einem Kunststoff verwendet wird, der im Bereich der ersten und zweiten Wellenlänge ($\lambda_1$, $\lambda_2$) nur eine geringe Eigenabsorption besitzt.

6. Verfahren nach einem der Ansprüche 1 bis 5, dadurch gekennzeichnet, dass zwei getrennte Infrarot-Teilstrahlungsbündel (10, 11) erzeugt werden, die unterschiedliche Wellenlängen ($\lambda_1$, $\lambda_2$) haben, und dass diese Teilbündel verschieden moduliert werden.

7. Vorrichtung zur Durchführung des Verfahrens nach einem der Ansprüche 1 bis 6, bestehend aus
- einer Infrarotstrahlungsquelle (3) zur Erzeugung eines Infrarotstrahlungsbündels (5), das aus Infrarotstrahlung einer ersten Wellenlänge ($\lambda_1$) und einer zweiten Wellenlänge ($\lambda_2$) besteht,
- einer Infrarotstrahlungsdetektoreinrichtung (4),
- einem im Infrarotstrahlengang zwischen der Quelle (3) und dem Detektor (4) positionierten Probeträger, sowie
- einer der Detektoreinrichtung (4) nachgeschalteten Divisionsschaltung (6) zur Bildung des Quotienten aus den ermittelten Absorptionswerten, wobei die Vorrichtung zur simultanen Messung der Absorptionswerte der Infrarotstrahlung bei den beiden unterschiedlichen Wellenlängen ($\lambda_1$, $\lambda_2$) ausgebildet ist,

dadurch gekennzeichnet, dass zur Bestimmung von Glucose im Serum oder im Harn die erste Wellenlänge ($\lambda_1$) im Bereich von 940 bis 950 cm$^{-1}$ und die zweite Wellenlänge ($\lambda_2$) im Bereich von 1090 bis 1095 cm$^{-1}$ liegt, wobei die Glucose-Konzentration dem bei der zweiten Wellenlänge ($\lambda_2$) gemessenen Absorptionswert proportional ist, während der bei der ersten Wellenlänge ($\lambda_1$) gemessene Absorptionswert praktisch unabhängig von dieser Konzentration ist.

8. Vorrichtung nach Anspruch 7, dadurch gekennzeichnet, dass als Infrarotstrahlungsquelle ein oder mehrere Laser vorgesehen sind.

9. Vorrichtung nach Anspruch 7, dadurch gekennzeichnet, dass die Infrarotstrahlungsquelle (3) mit einer Blende (9) zum Ausblenden eines ersten und zweiten Teilstrahlungsbündels (10, 11) versehen ist, dass im Strahlengang des ersten Teilstrahlungsbündels (10) ein erstes Interferenzfilter (12), das nur Infrarotstrahlung der ersten Wellenlänge ($\lambda_1$) durchlässt, und im Strahlengang des zweiten Teilstrahlungsbündels (11) ein zweites Interferenzfilter (13), das nur Infrarotstrahlung der zweiten Wellenlänge ($\lambda_2$) durchlässt, vorgesehen ist, und dass eine Strahlumlenk- und -zusammenführungsvorrichtung (14, 15) zum Vereinigen der beiden Teilstrahlungsbündel (10, 11) zu einem einzigen, die Probe (2) durchsetzenden Infrarotstrahlungsbündel (5) vorgesehen ist.

10. Vorrichtung nach Anspruch 7, dadurch gekennzeichnet, dass die Infrarotstrahlungsquelle (3) mit einer Blende (9) zum Ausblenden eines einzigen Infrarotstrahlungsbündels (5) versehen ist, in dessen Strahlengang ein Doppelband-Interferenzfilter (29) vorgesehen ist, das nur Infrarotstrahlung der ersten und zweiten Wellenlänge ($\lambda_1$, $\lambda_2$) durchlässt.

11. Vorrichtung nach Anspruch 9, dadurch gekennzeichnet, dass im Strahlengang des ersten und zweiten Teilstrahlungsbündels (10, 11) ein Chopper (16) zum Modulieren des ersten Teilstrahlungsbündels (10) mit einer ersten Chop-Frequenz ($f_1$) und zum Modulieren des zweiten Teilstrahlungsbündels (11) mit einer zweiten Chop-Frequenz ($f_2$) vorgesehen ist.

12. Vorrichtung nach Anspruch 11, dadurch gekennzeichnet, dass die Detektoreinrichtung (4) einen einzigen Infrarotstrahlungsdetektor (19) enthält, dem zwei parallele Lock-in-Verstärker (20, 21) nachgeschaltet sind, von denen der eine auf die erste Chop-Frequenz ($f_1$) und der andere auf die zweite Chop-Frequenz ($f_2$) abgestimmt ist, und dass die Ausgänge der Lock-in-Verstärker (20, 21) mit der Divisionsschaltung (6) verbunden sind.

## Claims

1. Method for determining glucose in serum or urine, in which the absorption of infrared radiation by a specimen is measured at two different wavelengths ($\lambda_1$, $\lambda_2$), characterized in that the absorption by the specimen is measured simultaneously at a first wavelength ($\lambda_1$) in the range of 940 to 950 cm$^{-1}$ and at a second wavelenght ($\lambda_2$) in a range of 1090 to 1095 cm$^{-1}$ and that the quotient of the absorption values measured at said two wavelengths ($\lambda_1$, $\lambda_2$) is formed, whereby the glucose-concentration is proportional to the absorption value measured at the second wavelength ($\lambda_2$), while the absorption value measured at the first wavelength ($\lambda_1$) is practically independent form said concentration.

2. Method according to claim 1, characterized in that the absorption at the both different wavelengths ($\lambda_1$, $\lambda_2$) is determined by means of transmission-spectroscopy.

3. Method according to claim 1, characterized in that the absorption at the both different wavelengths ($\lambda_1$, $\lambda_2$) is determined by means of reflection-spectroscopy.

4. Method according to one of claims 1 to 3, characterized in that the specimen (2) is provided as a smear or a film.

5. Method according to claim 4, characterized in that as specimen support (1) there is used a throw away or disposable specimen support constructed of a plastic which has only a slight absorption of its own in the range of the first and second wavelengths ($\lambda_1$, $\lambda_2$).

6. Method according to one of claims 1 to 5, characterized in that two individual infrared-component-radiation beams (10, 11) are generated, which have different wavelenghts ($\lambda_1$, $\lambda_2$), and that these component-beams are modulated differently.

7. Apparatus for carrying out the method according to one of claims 1 to 6, consisting of
- an infrared radiation source (3) for generating an infrared radiation beam (5), which consists of infrared radiation of a first wavelength ($\lambda_1$) and of a second wavelength ($\lambda_2$),
- an infrared radiation detector device (4),
- a specimen support positioned in the infrared radiation path between the source (3) and the detector (4), and
- a division circuit (6) arranged behind the detector device (4) which serves to form the quotient of the measured absorption values, whereby the apparatus is constructed to measure simultaneously the absorption values of the infrared radiation at said both different wavelengths ($\lambda_1$, $\lambda_2$), characterized that for determining glucose in serum or urine the first wavelength ($\lambda_1$) is in the range of 940 to 950 cm$^{-1}$ and the second wavelength ($\lambda_2$) is in the range of 1090 to 1095 cm$^{-1}$, whereby the glucose-concentration is proportional to the absorption value measured at the second wavelength ($\lambda_2$), while the absorption value measured at the first wavelength ($\lambda_1$) is practically independent from said concentration.

8. Apparatus according to claim 7, characterized in that one or more lasers are provided as infrared radiation source.

9. Apparatus according to claim 7, characterized in that the infrared radiation source (3) is provided with a diaphragm (9) for isolating a first and a second component radiation-beam, that in the path of the first component radiation-beam (10) a first interference filter (12) is provided, which passes only infrared radiation of the first wavelength ($\lambda_1$), that in the path of the second component radiation-beam (11) a second interference filter (13) is provided, which passes only infrared radiation of the second wavelength ($\lambda_2$), and that a beam deflecting and combining device (14, 15) is provided for combining said both component radiation-beams (10, 11) into a single infrared radiation beam (5) which passes through the specimen (2).

10. Apparatus according to claim 7, characterized in that the infrared radiation source (3) is provided with a diaphragm (9) for isolating a single infrared radiation-beam (5) within the ray path of which is provided a double-band interference filter (29) which passes only infrared radiation of the first and second wavelengths ($\lambda_1$, $\lambda_2$).

11. Apparatus according to claim 9, characterized in that a chopper (16) is provided in the ray path of the first and of the second component radiation-beam in order to modulate the first component radiation-beam (10) with a first chopper frequency ($f_1$) and to modulate the second component radiation-beam (11) with a second chopper frequency ($f_2$).

12. Apparatus according to claim 11, characterized in that the detector device (4) contains a single infrared radiation detector (19), behind which two lock-in amplifiers (20, 21) are connected, one of said amplifiers being tuned to the first chopper frequency ($f_1$), the other being tuned to the second chopper frequency ($f_2$), and that the outputs of the lock-in amplifiers (20, 21) are connected with the devision circuit (6).

## Revendications

1. Procédé pour le dosage du glucose dans le sérum ou l'urine, dans lequel on mesure à deux longueurs d'onde différentes ($\lambda_1$, $\lambda_2$) l'absorption d'un rayonnement infrarouge par un échantillon, caractérisé en ce que l'absorption à travers l'échantillon est mesurée simultanément à une première longueur d'onde ($\lambda_1$) dans l'intervalle 940 à 950 cm$^{-1}$ et à une deuxième longuer d'onde ($\lambda_2$) dans l'intervalle 1090 à 1095 cm$^{-1}$, et que l'on forme le quotient des valeurs d'absorption mesurées à ces deux longueurs d'onde ($\lambda_1$, $\lambda_2$), où la concentration du glucose est proportionnelle à la valeur d'absorption mesurée à la deuxième longueur d'one ($\lambda_2$), tandis que la valeur d'absorption mesurée à la première longueur d'one ($\lambda_1$) est pour ainsi dire indépendante de cette concentration.

2. Procédé selon la revendication 1, caractérisé en ce que l'absorption, aux deux longueurs d'onde différentes ($\lambda_1$, $\lambda_2$), est déterminée par spectroscopie par transmission.

3. Procédé selon la revendication 1, caractérisé en ce que l'absorption est déterminée aux deux longueurs d'onde différentes ($\lambda_1$, $\lambda_2$) par spectroscopie par réflexion.

4. Procédé selon l'une quelconque des revendications 1 à 3, caractérisé en ce que l'échantillon 2 est prévu sous la forme d'un frottis ou d'un film.

5. Procédé selon la revendication 4, caractérisé en ce qu'on utilise en tant que porte-échantillon (1) un porte-échantillon jetable ou à usage unique, en une matière plastique ne présentant dans le domaine de la première et de la deuxième longueurs d'onde ($\lambda_1$, $\lambda_2$) qu'une faible absorption propre.

6. Procédé selon l'une quelconque des revendications 1 à 5, caractérisé en ce que l'on forme deux faisceaux de rayonnement partiel infrarouge distinct (10, 11), qui ont des longueurs d'onde différentes ($\lambda_1$, $\lambda_2$), et que ces faisceaux partiels subissent des modulations différentes.

7. Appareillage pour la mise en œuvre du procédé selon l'une quelconque des revendications 1 à 6, comprenant
– une source de rayonnement infrarouge (3) destinée à produire un faisceau de rayonnement infrarouge (5) constitué d'un rayonnement infrarouge ayant une première longueur d'onde ($\lambda_1$) et une deuxième longueur d'onde ($\lambda_2$),
– un dispositif détecteur de rayonnement infrarouge (4),
– un porte-échantillon placé sur le trajet du rayonnement infrarouge entre la source (3) et le détecteur (4), et aussi

– un circuit diviseur (6), placé en aval du dispositif détecteur (4), destiné à former le quotient des valeurs déterminées pour l'absorption, l'appareillage étant constitué pour la mesure simultanée des valeurs d'absorption du rayonnement infrarouge aux deux longueurs d'onde différentes ($\lambda_1$, $\lambda_2$), caractérisé en ce que, pour doser le glucose dans le sérum ou l'urine, la première longueur d'one ($\lambda_1$) est comprise entre 940 et 950 cm$^{-1}$ et la deuxième longueur d'onde ($\lambda_2$) est comprise entre 1090 et 1095 cm$^{-1}$, la concentration du glucose étant proportionnelle à la valeur d'absorption mesurée à la deuxième longueur d'onde ($\lambda_2$), tandis que la valeur d'absorption mesurée à la première longueur d'onde ($\lambda_1$) est pour ainsi dire indépendante de cette concentration.

8. Appareillage selon la revendication 7, caractérisé en ce qu'il prévoit en tant que source de rayonnement infrarouge un ou plusieurs lasers.

9. Appareillage selon la revendication 7, caractérisé en ce que la source de rayonnement infrarouge (3) est pourvue d'un diaphragme (9) pour diaphragmer un premier et un deuxième faisceaux partiels (10, 11); que, sur le trajet du premier faisceau partiel (10), il est prévu un premier filtre interférentiel (12) ne laissant passer que le rayonnement infrarouge de la première longueur d'onde ($\lambda_1$) et, sur le trajet du deuxième faisceau partiel (11), un deuxième filtre interférentiel (13) ne laissant passer que le rayonnement infrarouge de la deuxième longueur d'onde ($\lambda_2$); et qu'il est prévu un dispositif de déviation et de recombinaison des rayons lumineux (14, 15), destiné à recombiner les deux faisceaux partiels (11) pour obtenir un faisceau de rayonnement infrarouge (5) unique tranversant l'échantillon.

10. Appareillage selon la revendication 7, caractérisé en ce que la source de rayonnement infrarouge (3) est pourvue d'un diaphragme (9) pour diaphragmer un faisceau de rayonnement infrarouge unique (5), sur le trajet duquel est prévu un filtre interférentiel à double bande passante (29), qui ne laisse passer que le rayonnement infrarouge de la première et de la deuxième longueurs d'onde ($\lambda_1$, $\lambda_2$).

11. Appareillage selon la revendication 9, caractérisé en ce qu'il est prévu sur le trajet du premier et du deuxième faisceaux de rayonnement partiel (10, 11) un hacheur (16) destiné à moduler le premier faisceau de rayonnement partiel (10) selon une première fréquence de hachage ($f_1$) et à la modulation du deuxième faisceau de rayonnement partiel (11) selon une deuxième fréquence de hachage ($f_2$).

12. Appareillage selon la revendication 11, caractérisé en ce que le dispositif détecteur contient un détecteur de rayonnement infrarouge unique (19), en aval duquel sont montés deux amplificateurs de blocage en parallèle (20, 21), dont l'un est accordé à la première fréquence de hachage ($f_1$) et l'autre à la deuxième fréquence de hachage ($f_2$); et que les sorties des amplificateurs de blocage (20, 21) sont connectées au circuit diviseur (6).

Fig.1

Fig.2

Fig.3

Fig.4

Fig.5

Fig. 6

Fig.7

Fig. 8

0 034 156

Fig.9

Fig.10

Fig.11

$\Delta \lambda \leqq 1\% \, (\lambda_2)$

$\Delta \lambda \leqq 1\% \, (\lambda_1)$

# Fig.12